# EUROPEAN PATENT APPLICATION

(11) **EP 1 184 000 A1**
(43) Date of publication of application: **06.03.2002**
(21) Application number: 00830593.0
(22) Date of filing: 31.08.2000
(51) Int. Cl.: A61B 17/64

(54) **External fixator for stabilizing bone fractures**

(71) Applicant: ORTHOFIX S.r.l., 37012 Bussolengo (VR) (IT)
(72) Inventor: Venturini, Daniele, 37064 Povegliano Veronese (VR) (IT)
(74) Representative: Botti, Mario

(57) **Abstract**

In an external splint device for reducing bone fractures, being of the type which comprises at least two rod-like elements, such as a bone screw stem and a tie rod, and comprises a clamping component for clamping said rod-like elements tightly together, which has the advantage of being structurally simple and easy to handle, the clamping component comprises two identical clamps laid side-by-side, each clamp having two jaws for clamping a respective rod-like element, and comprises a screw led through the jaws for clamping them between a head of the screw and a clamping nut threaded onto the screw.

## Description

### Field of the Invention

This invention broadly relates to an external splint device for reducing bone fractures in orthopedic surgery, which device may be of the so-called hybrid type comprising a ring and a unilateral axial splint.

More particularly, the invention relates to an external splint device for reducing bone fractures, which comprises at least two rod-like elements, such as a bone screw stem and a tie rod, and comprises a clamping component for fastening said rod-like elements tightly together.

It is a recognized fact that the general structure of an external splint device, including all the parts thereof, should form a stable and relatively non-distortable whole, so as to ensure that a broken bone, to which such structure is applied, can be firmly held in the correct position from the time the splint is applied to the final formation of the callus and, ultimately, to the fracture reduction.

### Prior Art

Clamping components have been known and extensively used to fasten together the component parts of an external splint device structure as above, in particular to fasten together such rod-like elements as a stem of a bone screw and the tie rod, these clamping components being fairly complicated constructions made up of several parts.

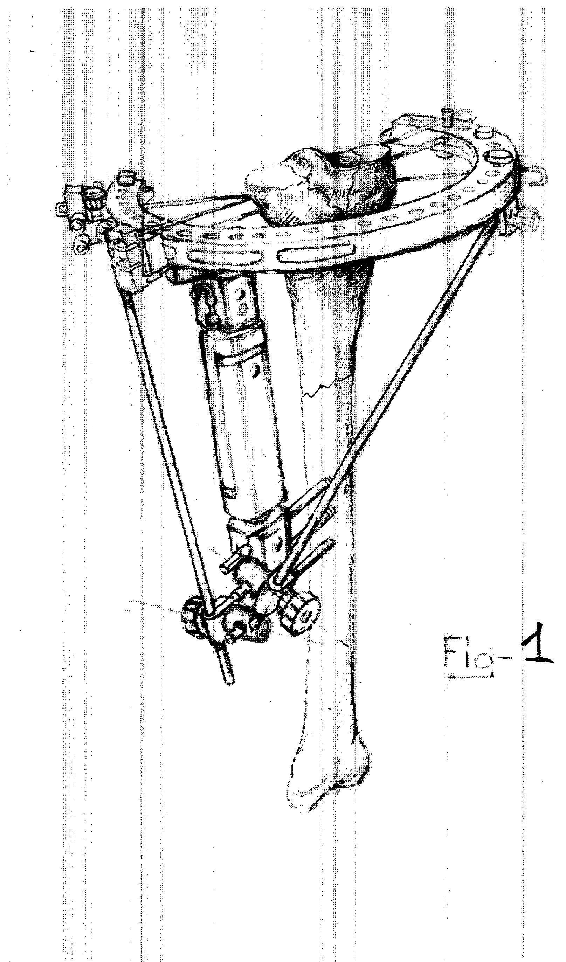

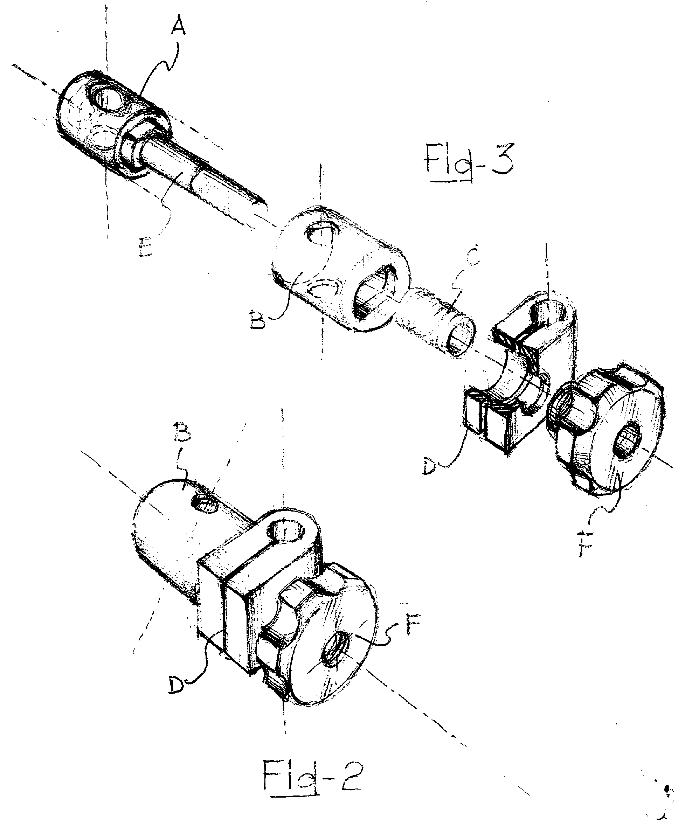

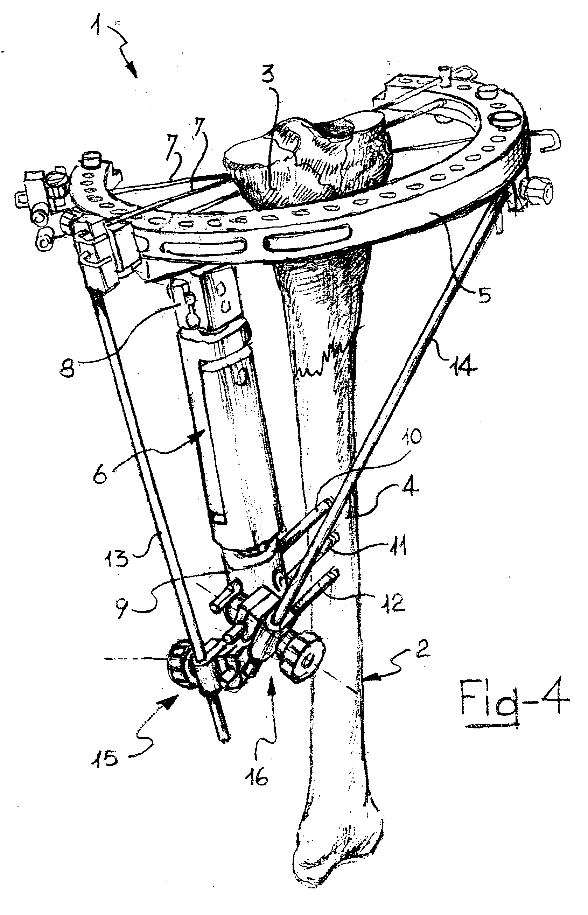

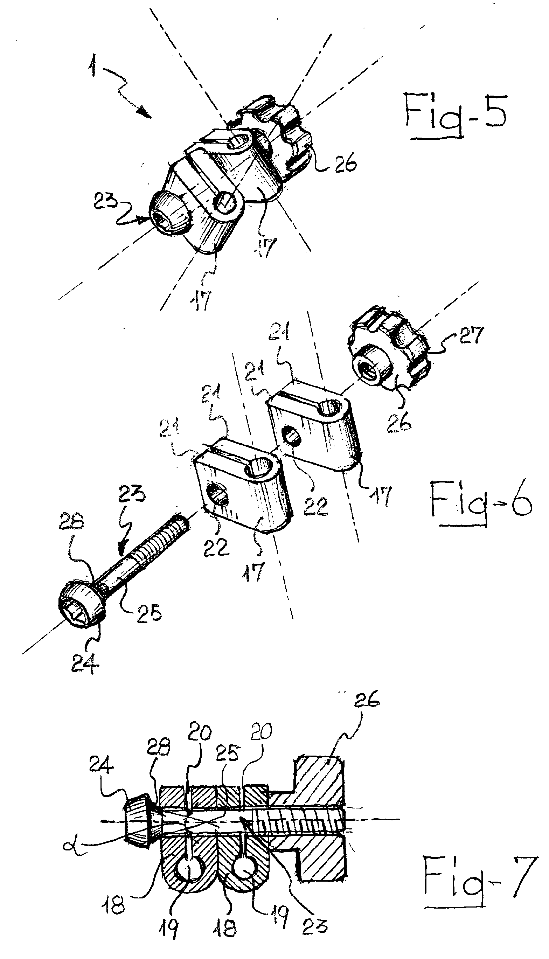

One example of a conventional clamping component is shown in Figures 1, 2 and 3. This clamping component has one cylindrical element captured between two sleeves A and B which are telescopically coupled to each other and constantly stressed by a spring C to reciprocal positions in which the cylindrical element is captured, and has the other cylindrical element clamped in a clamp D operated by means of a clamping screw V which is mounted to a sleeve (A) and acts against the contrast of the other, and by means of a clamping nut F.

As said before, this structure is a complicated one also because it is made of many parts; in fact, it comprises five all different parts.

The underlying problem of this invention is to provide a device as specified above with appropriate structural and functional features to overcome the aforementioned disadvantage of the prior art.

### Summary of the Invention

The invention relates to an external splint device as specified above, which device is characterized in that the clamping component comprises two clamps laid side-by-side and each having two jaws adapted to be tightly clamped around a respective rod-like element, and comprises a screw led through the jaws to clamp them between a screw head and a clamping nut threaded onto the screw.

Further features and advantages of the external splint device according to the invention will be apparent from the following description of a preferred embodiment thereof, given by way of non-limitative example with reference to the accompanying drawings.

### Brief Description of the Drawings

Figure 1 shows a perspective view of an external splint device according to the prior art.

Figure 2 shows a perspective view, drawn to an enlarged scale, of a detail of the device of Figure 1.

Figure 3 shows a perspective view with removed part of the detail shown in Figure 2.

Figure 4 shows a perspective view of an external splint device according to this invention.

Figure 5 shows a perspective view, drawn to an enlarged scale, of a detail of the device shown in Figure 4.

Figure 6 shows a perspective view with removed parts of the detail shown in Figure 5.

Figure 7 shows a sectional view of the detail shown in Figure 5.

### Detailed Description of the Invention

With reference to the accompanying drawings, an external splint device intended for application to a long broken bone, such as a tibia 2, between a proximal end 3 and a middle portion 4 thereof, in order to reduce the bone fracture, is generally shown at 1.

The splint device 1 is of the type called hybrid, and comprises a ring 5 and a unilateral external splint 6.

The ring 5 encircles the proximal end 3 and is held thereto by wires 7.

The unilateral external splint 6 has an upper end 8 fastened to the ring 5 and a lower end 9 fastened to the middle portion 4 of the bone 2 by means of three bone screws 10, 11 and 12 which extend approximately parallel to one another and perpendicularly to the bone axis.

The device 1 is stiffened by a pair of cylindrical rods 13 and 14 which are arranged to rigidly connect two diametrically opposed spots of the ring 5 and the lower end 9 of the unilateral external splint 6.

In particular, the rod 13 has an upper end fastened to the ring 5 and a lower end fastened to the screw 11, while the rod 14 has an upper end fastened to the ring 5 and a lower end fastened to the screw 12.

The lower end of the rod 13 and the screw 11 are fastened together by a clamping component 15. An identical clamping device 16 is used for clamping together the lower end of the rod 14 and the screw 12.

The clamping component 15, comprising two identical clamps 17 laid side-by-side, will now be described in greater detail.

Each clamp 17 comprises a block 18 which is drilled with a hole 19 intended to receive therein with limited clearance a rod-like element to be clamped, and which is slit at 20 down to the hole 19. The hole 19 and slit 20 jointly define two jaws 21 in the block for clamping the rod-like element.

A hole 22, having a perpendicular axis to the axis of the hole 19 and not interfering with the hole 19, extends through the jaws 21. A screw 23, having a head 24 and a threaded stem 25, is inserted through the holes 22 of the two side-by-side clamps 17 and serves to clamp their jaws 21 between the head 24 of the screw 23 and a clamping nut 26 threadably fitting-onto the threaded stem 25 of the screw 23.

The clamping nut 26 is advantageously formed with a knurled periphery 27 to be easily maneuvered by hand.

It should be noted that the screw 23 has a conical portion 28 of short length and slight taper, e.g. a taper angle α=20°, which lies next to the head 24, for frictionally engaging the hole 22 of a jaw 21 of a clamp 17 as the clamping nut 26 is clamped.

Advantageously, both clamps are formed of a transparent material to X-radiation, such as a plastics matrix of polyether ketone.

In use, the clamping of the nut 26 implies the clamping together of each of the two clamps 17 which are side by side positioned on the respective rod-like elements, that is the clamping together of the lower end of the rod 13 and the screw 11 as relates to the clamp device 15, and the lower end of the rod 14 and the screw 12 as relates to the clamp device 16, are clamped together.

As the nut 26 is clamped, the screw 23 and clamp 17 become fast together upon the conical portion 28 frictionally engages in the hole 22 of one jaw 21, the two side-by-side clamps 17 being concurrently made fast together by friction. In this way, the clamping nut can be tightened down using one hand, with no need to hold the clamps against undesired rotation with the other hand.

This equally applies to the loosening operation, when the clamping nut is turned contrariwise with one hand and there is no need to use the other hand to detain the clamps from rotating.

The major advantage of the invention is its simple construction, an important factor for a component which is to be manufactured in large volumes. In fact, the clamping component is made of four parts, of which two are identical, instead of five different parts.

A further advantage is the fact that the clamping component requires for its operation, that is during the clamping phase, the use of only one hand. In fact when the clamping nut is maneuvered, the screw is not led to rotation, because such a screw is detained from turning by the frictional force applied by the cone, which frictional force increases with clamping.

Furthermore it should be noted, that the clamping component can be used in combination with X-ray apparatus without having its efficiency limited, since it is made for the most part of a material transparent to X-radiation such as a plastics matrix of polyether ketone.

## Claims

1. An external splint device for reducing bone fractures, comprising at least two rod-like elements, such as a bone screw stem and a tie rod, and comprising a clamping component for clamping said rod-like elements tightly together, **characterized in that** the clamping component comprises two clamps laid side-by-side and each having two jaws for tightly clamping around a respective rod-like element, and comprises a screw led through the jaws for clamping them between a head of the screw and a clamping nut threaded onto the screw.

2. An external splint device according to Claim 1, **characterized in that** the screw has a conical portion lying close to the head for frictionally engaging one of said jaws as the clamping nut is clamped.

3. An external splint device according to Claim 1, **characterized in that** the two clamps are identical.

4. An external splint device according to Claim 1, **characterized in that** both clamps are formed of a material transparent to X-radiation, such as a plastics matrix of polyether ketone.

5. A clamping component for clamping two rod-like elements of an external splint device for stabilizing bone fractures as claimed in Claims 1 to 4.
